(19) Europäisches Patentamt / European Patent Office / Office européen des brevets

(11) **EP 2 807 932 B1**

(12) **FASCICULE DE BREVET EUROPEEN**

(45) Date de publication et mention de la délivrance du brevet:
**23.11.2016 Bulletin 2016/47**

(21) Numéro de dépôt: **14170704.2**

(22) Date de dépôt: **30.05.2014**

(51) Int Cl.:
*A61K 9/50* (2006.01)   *A23P 10/30* (2016.01)
*A61K 35/747* (2015.01)   *C12N 1/04* (2006.01)
*A61K 47/22* (2006.01)   *C12N 1/20* (2006.01)
*C12N 9/96* (2006.01)

(54) **Procédé d'encapsulation de molécules bioactives dans des matrices laitières et microparticules obtenues**

Einkapselungsverfahren von bioaktiven Molekülen in Milchmatrizen, und so erhaltene Mikropartikel

Method for encapsulating bioactive molecules in dairy matrices and microparticles obtained

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR

(30) Priorité: **31.05.2013 FR 1355029**

(43) Date de publication de la demande:
**03.12.2014 Bulletin 2014/49**

(73) Titulaire: **Université de Lorraine**
**54052 Nancy Cedex (FR)**

(72) Inventeurs:
• **Burgain, Jennifer**
  **54600 VILLERS-LES-NANCY (FR)**
• **Gaiani, Claire**
  **54600 VILLERS LES NANCY (FR)**
• **Jeandel, Carole**
  **54740 GERBECOURT ET HAPLEMONT (FR)**
• **Ghoul, Mohamed**
  **54000 NANCY (FR)**
• **Scher, Joël**
  **54520 LAXOU (FR)**

(74) Mandataire: **Pontet Allano & Associes**
**Parc Les Algorithmes, Bâtiment Platon**
**CS 70003 Saint-Aubin**
**91192 Gif-sur-Yvette Cedex (FR)**

(56) Documents cités:

• **Ivana Lisová ET AL: "Emulsion Encapsulation of Bifidobacterium animalis subsp. lactis BB12 with the Addition of Lecithin", Czech J. Food Sci, vol. 31, no. 3 1 mai 2013 (2013-05-01), pages 270-274, XP055099208, Extrait de l'Internet: URL:http://www.agriculturejournals.cz/publ icFiles/104962.pdf [extrait le 2014-01-29]**
• **HEIDEBACH T ET AL: "Influence of casein-based microencapsulation on freeze-drying and storage of probiotic cells", JOURNAL OF FOOD ENGINEERING, BARKING, ESSEX, GB, vol. 98, no. 3, 1 juin 2010 (2010-06-01), pages 309-316, XP026910919, ISSN: 0260-8774, DOI: 10.1016/J.JFOODENG.2010.01.003 [extrait le 2010-01-07]**
• **ION TITAPICCOLO G ET AL: "Modification to the renneting functionality of casein micelles caused by nonionic surfactants", JOURNAL OF DAIRY SCIENCE, AMERICAN DAIRY SCIENCE ASSOCIATION, US, vol. 93, no. 2, 1 février 2010 (2010-02-01), pages 506-514, XP026954930, ISSN: 0022-0302 [extrait le 2010-02-01]**
• **HEIDEBACH T ET AL: "Microencapsulation of probiotic cells by means of rennet-gelation of milk proteins", FOOD HYDROCOLLOIDS, ELSEVIER BV, NL, vol. 23, no. 7, 1 octobre 2009 (2009-10-01), pages 1670-1677, XP026145859, ISSN: 0268-005X, DOI: 10.1016/J.FOODHYD.2009.01.006 [extrait le 2009-01-19]**

Remarques:
Le dossier contient des informations techniques présentées postérieurement au dépôt de la demande et ne figurant pas dans le présent fascicule.

**Description**

La présente invention a pour objet un procédé d'encapsulation de molécules bioactives dans des matrices laitières, les microparticules obtenues par ledit procédé et leur utilisation dans les domaines cosmétiques, alimentaires et pharmaceutiques.

Aujourd'hui les consommateurs s'intéressent de plus en plus aux aliments pouvant apporter un bénéfice sur leur santé. C'est pourquoi il y a un intérêt grandissant pour les produits à base de probiotiques.

En 2001, l'Organisation mondiale de la Santé (OMS) et l'Organisation des Nations unies pour l'alimentation et l'agriculture (FAO) ont donné une définition officielle des probiotiques qui sont des « micro-organismes vivants qui, lorsqu'ils sont ingérés en quantité suffisante, exercent des effets positifs sur la santé, au-delà des effets nutritionnels traditionnels ».

Ces micro-organismes vivants, bactéries ou levures, aident à la digestion des fibres, stimulent le système immunitaire, préviennent ou traitent la diarrhée, diminuent le cholestérol, améliorent la tolérance au lactose ou préviennent certains cancers. Toutefois ces effets sont fonction des souches utilisées.

Parmi les microorganismes utilisés comme probiotiques, on retrouve souvent

- des bactéries lactiques (LAB), notamment "Bifidobacterium spp." plus particulièrement les espèces *Bifidobacterium bifidum (bifidus), Bifidobacterium lactis, Bifidobacterium longum, Bifidobacterium breve, Lactobacillus spp.* plus particulièrement *Lactobacillus reuteri, Lactobacillus acidophilus, Lactobacillus casei, Lactobacillus plantarum* et *Lactobacillus rhamnosus,*
- des bactéries non lactiques notamment *Escherichia coli* et
- des levures comme par exemple *Saccharomyces cerevisiae et Saccharomyces boulardii.*

Pour que les probiotiques aient un effet bénéfique sur la santé, il faut que plusieurs conditions soient réunies :

- qu'ils soient vivants (ou lyophilisés),
- que les bonnes souches soient sélectionnées pour l'effet recherché,
- que les souches aient montré leur résistance à l'acidité gastrique et à la bile,
- que les cures soient d'au moins 10 jours par mois et
- que la démonstration de leur bénéfice ait été faite tant chez l'être humain sain que chez le malade.

Surtout, pour être efficaces les probiotiques doivent impérativement parvenir vivants dans le côlon et en nombre suffisant. Ils ne doivent en effet pas être dégradés suite à leur passage dans l'estomac et doivent être capables de résister à l'acidité gastrique et au suc du pancréas.

Des concentrations efficaces de probiotiques dans les aliments ont été définies, qui sont non seulement fonction du type de probiotique et du type d'aliment, mais aussi fonction du pays concerné. Ainsi un minimum de $10^6$ UFC/g (Unité formant colonies/g) a été fixé pour les bifidobactéries dans les produits laitiers pour les pays de MERCOSUR (Argentine, Bolivie, Brésil, Chili, Paraguay et Uruguay), alors qu'au Japon un minimum de $10^7$ UFC/g a été recommandé dans les produits laitiers frais. Au niveau européen, la réglementation a fixé des concentrations minimales de $10^9$ UFC/g pour certains microorganismes utilisés en tant qu'additifs alimentaires.

Le maintien de la viabilité et de la fonctionnalité des bactéries probiotiques jusqu'à ce qu'elles atteignent le tube digestif est donc primordial.

Or, la survie des bactéries probiotiques libres introduites dans les produits alimentaires est faible en raison des conditions défavorables rencontrées pendant le stockage et le passage dans l'estomac.

C'est pourquoi la microencapsulation est depuis longtemps utilisée car c'est une technologie puissante qui permet de protéger les cellules bactériennes et d'augmenter la survie des bactéries probiotiques durant le stockage puis le transit gastrique.

C'est un processus physicochimique qui permet de piéger une substance ou une cellule vivante dans un matériau, afin de produire des microparticules ou des microcapsules dont le diamètre peut aller de quelques nanomètres à quelques millimètres. Ce procédé permet de former une barrière protectrice pour composés encapsulés comme les probiotiques. Les systèmes encapsulés peuvent être de différents types, soit de type réservoir (a), soit de type matrice enrobée (b), soit de type matriciel (c),

**(a)** **(b)** **(c)**

Cette technique est applicable non seulement aux probiotiques mais aussi à d'autres molécules bioactives fragiles qui doivent être protégées de l'environnement ou véhiculées vers une cible spécifique et ce, dans des domaines aussi diverses que, notamment, le domaine pharmaceutique, le domaine cosmétique, le domaine alimentaire et le domaine textile.

Dans le cas des probiotiques, les matériaux utilisés doivent être de qualité alimentaire. Il s'agit le plus souvent de polymères naturels, tels que l'alginate, les carraghénanes, la gomme gellane, le chitosan, la gélatine, l'amidon ou des protéines (notamment celles du lait). Les technologies utilisées pour l'encapsulation sont par exemple l'atomisation, l'émulsification ou l'extrusion (Burgain J. et al. Journal of Food Engineering, (2011), 104, 467-483).

Parmi les matériaux utilisés pour encapsuler les probiotiques, les produits laitiers ont démontré un avantage qui pourrait être attribué au pouvoir tampon des protéines laitières et à leur capacité à former un réseau suite à la coagulation (Livney Y.D. in Colloid & Interface Science, 2010, 15, 73-83). Par ailleurs, ces protéines sont autorisées, sans restriction par la législation, dans les produits alimentaires.

Heidebach T. et al. (International Dairy Journal, 2009, 19, 77-84) décrivent une méthode d'encapsulation de cellules probiotiques dans des microcapsules de caséine de qualité alimentaire. Cette méthode, en circuit ouvert, est basée sur la gélification catalysée par une transaminase, de suspensions de caséine contenant des probiotiques. Seule l'huile de tournesol est utilisée comme matière grasse et c'est une encapsulation à l'échelle laboratoire, sur de petites quantités. Toutefois, cette méthode ne permet de produire que de très faibles quantités de microparticules à une échelle laboratoire.

Lisova et al. (Czech Journal of Food Science, 2013, 31, 270-274) décrit une méthode d'encapsulation de cellules probiotiques en utilisant le lait écrémé, présure, l'huile de tournesol et lécithine.

Le brevet EP 1876905 décrit une méthode d'encapsulation d'acides gras sensibles à l'oxydation à l'aide d'une émulsion huile dans eau (l'huile étant la molécule bioactive et la phase aqueuse contenant des caséinates de sodium, la solution protéique) à des températures supérieures à 70 °C ; cette émulsion est ensuite séchée pour donner une matrice.

Les inventeurs ont également décrit un procédé à l'échelle du laboratoire qui met en oeuvre des protéines laitières et montré qu'il était possible de réaliser une encapsulation de probiotiques avec de bons rendements ; les microcapsules obtenues permettent d'augmenter la survie des probiotiques durant le stockage ainsi que le transit gastrique (présentation orale au XIX International Conférence on Bioencapsulation, Amboise, France, 5-8 octobre 2011). Toutefois ce procédé est réalisé en circuit ouvert, et en l'absence d'agitation de la phase enzymatique et n'est pas transposable à l'échelle industrielle.

En général, alors qu'au niveau du laboratoire les résultats sont prometteurs, les technologies mises en oeuvre posent souvent des difficultés de mise à l'échelle et sont difficilement transposables à l'échelle industrielle.

En outre, réduire la taille des microparticules reste un défi majeur, car il faut éviter que les microparticules affectent la texture et les propriétés sensorielles d'un produit alimentaire, cosmétique ou pharmaceutique dans lequel elles sont incorporées.

Aussi la présente invention a-t-elle pour objectif de fournir un procédé d'encapsulation de molécules bioactives permettant une résistance à l'environnement gastrique, et une libération contrôlée au niveau d'une cible spécifique, notamment au niveau intestinal et permettant de disposer de particules dont la structure (taille, forme..) et les propriétés rhéologiques font qu'elles n'affectent pas la texture, ni les propriétés sensorielles des produits qui les contiennent. Ce procédé est utilisable à l'échelle industrielle, permet d'encapsuler tout type de molécules bioactives et met en oeuvre une matrice composée de protéines de lait et de composants de qualité alimentaire.

La présente invention a également pour but de fournir des microparticules permettant d'augmenter la survie durant le stockage puis le transit gastrique des molécules bioactives qu'elles contiennent.

Aussi la présente invention a pour objet un procédé d'encapsulation de molécules bioactives par des protéines laitières choisies dans le groupe comprenant :

- de la caséine micellaire seule,
- de la caséine micellaire associée à des protéines laitières solubles natives,
- de la caséine micellaire associée à des protéines laitières solubles dénaturées, et
- de la caséine micellaire associée à des protéines laitières solubles natives et à des protéines laitières solubles dénaturées,

le dit procédé étant mis en oeuvre en circuit fermé et comprenant les étapes suivantes :

a. la mise en contact d'une solution desdites protéines laitières présentant un pH compris entre 4,8 et 8 et contenant au moins une molécule bioactive à encapsuler, avec la chymosine, à une température comprise entre 1 et 10 °C, avantageusement 3 et 5 °C, jusqu'à ce qu'au moins 85 % du caséinomacropeptide soit clivé,

b. l'addition progressive de la solution protéique obtenue à l'étape a) à une matière grasse en présence d'un agent tensioactif à une température comprise entre 1 et 10 °C, avantageusement entre 3 et 5°C sous agitation comprise entre 100 et 1400 rpm, jusqu' à formation de gouttelettes et obtention d'une émulsion. La vitesse d'agitation à appliquer est fonction de la taille des particules souhaitée,

c. l'application d'une rampe de température allant de 1 à 60 °C, avantageusement de 5 à 40 °C, et

d. une phase de séparation des microparticules formées.

Conformément à l'invention, le procédé est réalisé sans qu'il soit nécessaire d'ajouter du $CaCl_2$ puisque les protéines mises en oeuvre coagulent sans cet ajout.

Au sens de la présente invention, on entend par caséine micellaire un assemblage de caséines $\alpha$, $\beta$, et $\kappa$ dont le diamètre moyen est de 120 nm. L'obtention des caséines micellaires se fait par des techniques de filtration membranaire du lait écrémé : microfiltration (pour purifier et ensuite séparer les caséines micellaires des protéines solubles, du lactose et des minéraux) et diafiltration (pour concentrer les caséines micellaires). Ces techniques sont connues de l'homme du métier.

Au sens de la présente invention, on entend par protéines laitières solubles natives: la $\beta$-lactoglobuline, l'$\alpha$-lactalbumine, les immunoglobulines (10%), l'albumine de sérum bovin et la lactoferrine. L'obtention des protéines solubles se fait par des techniques de filtration membranaires du lait écrémé : microfiltration (pour purifier et ensuite séparer les protéines solubles, le lactose et les minéraux des caséines micellaires), une ultrafiltration tangentielle (pour éliminer les minéraux et le lactose) et finalement une diafiltration (pour concentrer les protéines solubles). Ces techniques sont connues de l'homme du métier.

Au sens de la présente invention, on entend par protéines laitières solubles dénaturées, les protéines obtenues par dénaturation des protéines laitières solubles natives, notamment par chauffage.

Au sens de la présente invention on entend par « circuit fermé » un système complètement isolé de l'extérieur, ce qui empêche toute contamination externe.

Selon la présente invention, la matière grasse peut être toute matière grasse classiquement utilisée pour des procédés d'encapsulation sous réserve qu'elle soit fluide aux températures auxquelles le procédé est mis en oeuvre, c'est-à-dire entre 1 et 60 °C. Elle peut être de nature alimentaire pour des applications alimentaires ou de nature non alimentaire pour d'autres types d'applications (notamment textile).

La réaction de coagulation du lait peut être divisée en trois processus cinétiques principaux. Le premier est la réaction enzymatique qui consiste en une hydrolyse de la caséine-$\kappa$, le second est un processus non-enzymatique de floculation des micelles de caséine et le troisième consiste en la formation de liaisons au sein du gel.

La chymosine est une enzyme utilisée en fromagerie et est capable de libérer le caséinomacropeptide (CMP) par clivage de la liaison Phe105-Met106 de la caséine-K, située à la surface des micelles de caséine dans le lait. La libération de ce peptide entraîne une diminution de la charge nette négative et une augmentation de l'hydrophobicité de la para-K-caséine, ce qui va conduire à l'agrégation des micelles de caséine.

Toutefois, si la chymosine est mise au contact du lait à faible température, la caséine-$\kappa$ est clivée mais les micelles ne coagulent pas ou très peu, et ce n'est qu'en augmentant la température que la formation du gel sera rendue possible. C'est ce découplage de la phase primaire enzymatique et de la phase secondaire non-enzymatique qui a été exploité pour la mise au point d'un système d'encapsulation où la coagulation a été déclenchée au moment adéquat par une augmentation de la température.

Conformément à l'invention, lors de l'étape a), le CMP est libéré de la partie C-terminale de $\kappa$-caséine suite à l'action de la chymosine. En effet, il est nécessaire que l'étape a) ait atteint ce stade pour que l'étape c), c'est-à-dire la coagulation des protéines suite à l'augmentation de la température, puisse débuter. L'étape a) est appelée phase enzymatique.

Conformément à l'invention, l'agitation minimale à l'étape b) doit permettre un cisaillement suffisant pour la formation de gouttelettes. En revanche il est impératif d'éviter la dénaturation des molécules bioactives à encapsuler suite à un trop fort cisaillement. Aussi, l'agitation maximale tient compte de la vulnérabilité des molécules bioactives face au cisaillement, de la viscosité de la matière grasse (elle-même dépendante de la température) et de la nature du mobile d'agitation. Une vitesse d'agitation comprise entre 100 rpm et 1400 rpm satisfait à ces exigences. L'homme du métier saura adapter la vitesse d'agitation à ces différents paramètres à la lumière de ses connaissances générales.

La durée de l'étape a) dépend du rapport enzyme/substrat mais également de paramètres tels que la température et le pH. L'homme du métier, saura, à la lumière de ses connaissances générales, choisir la durée en fonction de ces différents paramètres.

La durée de l'addition de la solution protéique, obtenue lors de la phase enzymatique, à l'étape a) doit être suffisamment courte pour permettre la formation de gouttelettes au fur et à mesure de l'addition de la phase enzymatique à une matière grasse. Avantageusement la durée sera de 4 à 16 L/heure, notamment comprise entre 5 et 10 L/heure.

Le type d'agent tensioactif sera choisi parmi ceux couramment utilisés par l'homme du métier dans le domaine. On peut citer, à titre d'exemple, le Tween® 80 et le Span® 20, à une concentration de 1 % p/v par rapport à l'huile de tournesol utilisée comme matière grasse. Il devra être de grade alimentaire ou pharmaceutique en fonction de l'application.

Les rapports entre la quantité d'enzyme et la quantité de protéines laitières dépendent de la structure voulue du produit final ; l'homme du métier saura les adapter à la lumière de ses connaissances générales. A titre d'exemple on peut utiliser un rapport de 0,05 à 0,4 IMCU/g de protéines laitières pour l'enzyme Naturen® de chez Hansen.

Conformément à l'invention, la rampe de température va de la température d'incubation enzymatique (entre 1 et 10°C) jusqu'à 60°C, la température d'action optimale de la chymosine étant de 40°C. Une température trop élevée peut entraîner :

- une dénaturation de la chymosine, ou
- une destruction de la molécule bioactive ou
- une dénaturation des protéines.

La durée de cette rampe peut varier en fonction du produit final souhaité et du matériel utilisé. Si le transfert de chaleur est efficace au sein de l'installation la durée peut être réduite. Toutes ces adaptations sont à la portée de l'homme du métier.

L'étape d) de séparation, peut être réalisée par toute technique connue de l'homme du métier, notamment par filtration ou centrifugation.

Dans un mode de réalisation avantageux de l'invention, les molécules bioactives s'entendent comme des molécules biologiquement actives comme par exemple des médicaments, des produits cosmétiques et des compléments alimentaires, et sont choisies dans le groupe comprenant notamment :

- les composés phytochimiques : flavonoïdes, catéchines, phytoestrogènes (isoflavones/lignanes), anthocyanidines, tanins (proanthocyanidines), lycopène (caroténoïde), phytostérols (sitosterol/ stanol ester), fibres (insolubles et solubles), β-Glucane (fibre), lutéine (caroténoïde)
- les lipides bioactifs : acides gras ω-3, acide linoléique conjugué,
- les protéines et peptides bioactifs : lactoferrine, peptides bioactifs, enzymes (lactase...),
- les prébiotiques : fructo-oligosaccharides (FOS), sucres alcools,
- les bactéries probiotiques : bactéries lactiques, bifidobactéries,
- les vitamines : acide folique, tocophérols, α-carotène, β-carotène,
- les minéraux : calcium.

Le procédé selon l'invention est particulièrement intéressant pour l'encapsulation des molécules bioactives alimentaires, hydrophiles, amphiphiles ou hydrophobes, notamment pour l'encapsulation des probiotiques.

Conformément au procédé de l'invention, toutes ces molécules bioactives peuvent être encapsulées seules ou en mélange d'au moins deux d'entre elles. On peut encapsuler, par exemple, un prébiotique avec un probiotique ou une enzyme et une vitamine ou un probiotique avec une vitamine sous réserve qu'il n'y ait pas d'interactions négatives entre les différentes molécules.

Dans un mode de réalisation avantageux de l'invention, le procédé comprend après l'étape d), une étape de récupération des microparticules formées et éventuellement une étape de séchage desdites microparticules pour faire une poudre.

La récupération des microparticules est réalisée par filtration, centrifugation ou toute autre technique de séparation connue de l'homme du métier.

Le séchage peut être réalisé par n'importe quelle technique connue de l'homme du métier, notamment par une étape de lyophilisation.

Dans un mode de réalisation avantageux du procédé selon l'invention, la matière grasse est choisie dans le groupe comprenant :

- les huiles fluides entre 1 et 60 °C, en particulier des huiles végétales, notamment l'huile de tournesol,
- la matière grasse laitière fractionnée fluide entre 1 et 60 °C et
- les matières grasses bénéfiques pour la santé, notamment celles contenant des acides gras polyinsaturés, comme par exemple les huiles riches en co3.

Dans un mode de réalisation avantageux de l'invention, cette matière grasse peut également avoir un rôle de stabilisateur vis-à-vis des molécules à encapsuler. En effet, une fine couche de matière grasse reste adsorbée à la surface des microparticules. Cette couche lipidique peut ainsi s'opposer à la migration de composé de l'intérieur de la microparticule vers l'extérieur mais également du milieu environnant vers l'intérieur de la microparticule.

Dans un mode de réalisation avantageux du procédé selon la présente invention, la chymosine est utilisée à une concentration comprise entre 0,01 et 0,5 IMCU/g de protéines laitières, avantageusement entre 0,05 à 0,4 IMCU/g de protéines laitières (IMCU = International Milk Clotting Unit).

Dans un autre mode de réalisation avantageux de l'invention, de la mucine est ajoutée aux protéines laitières, à raison de 0 à 20 % de mucine par rapport au poids de protéine laitière. Tout type de mucine peut être utilisé, notamment la mucine laitière ou la mucine porcine.

Dans un autre mode de réalisation avantageux de l'invention, dans la solution de protéines laitières, la caséine micellaire représente entre 70 et 100 % en poids de la solution, les protéines laitières solubles natives représentent entre 0 et 30 % en poids de la solution et les protéines laitières solubles dénaturées représentent entre 0 et 30 % en poids de la solution.

Ainsi conformément à l'invention la solution de protéines laitières peut contenir soit uniquement de la caséine micellaire, soit de la caséine micellaire et des protéines laitières solubles natives, soit de la caséine micellaire et des protéines laitières solubles dénaturées, soit de la caséine micellaire, des protéines laitières solubles natives et des protéines laitières solubles dénaturées.

Dans un mode de réalisation avantageux de l'invention, la fraction volumique (φ), qui représente le rapport de la phase dispersée/phase totale,

$$\varphi = \frac{\text{Volume de protéine laitière}}{\text{Volume de protéine laitière + volume de matière grasse}}$$

est inférieur ou égal à 0,2.

Une fraction volumique supérieure à 0,2 entraine la formation d'un système concentré qui ne permet pas de produire efficacement des microparticules.

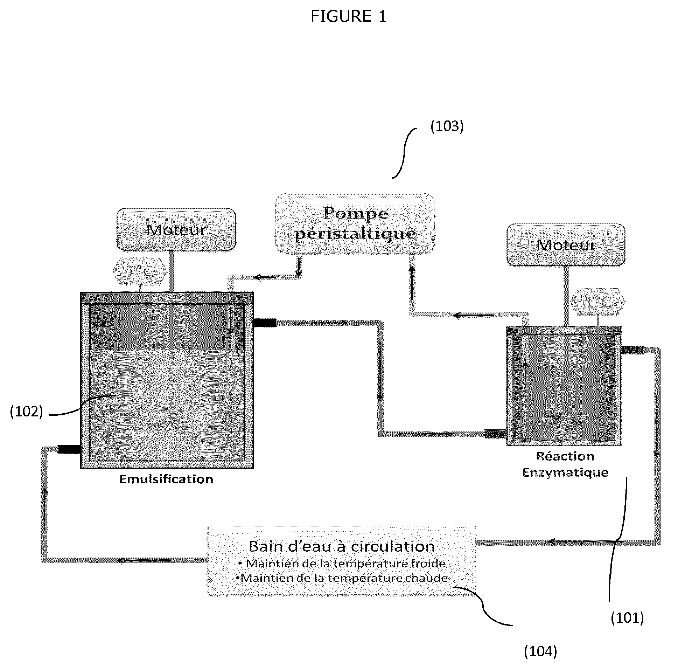

Le procédé de l'invention peut être mis en oeuvre par tout dispositif permettant de travailler en circuit fermé sous agitation. La figure 1 est une représentation schématique d'un exemple de dispositif fermé apte à mettre en oeuvre l'invention. Ce dispositif comprend :

- une enceinte fermée (101) sous agitation, dite enceinte pour la réaction enzymatique, dans laquelle est réalisée la réaction enzymatique, c'est-à-dire l'étape a),
- une enceinte fermée (102) sous agitation dite enceinte d'émulsification, dans laquelle est réalisée l'étape d'émulsification, c'est-à-dire les étapes b) et c),
- des moyens (103), qui permettent d'amener la solution de protéines obtenues à l'étape a) dans l'enceinte (102) contenant la matière grasse,
- des moyens de régulation de température (104), des échangeurs efficaces munis des systèmes de régulation de température ou tout équipement connu de l'homme de l'art permettant le contrôle de la température à un point consigne ou réaliser un profil de profil programmable, pour, dans un premier temps, réfrigérer le système (enceinte pour la réaction enzymatique et enceinte d'émulsification) à une température d'environ 5°C puis dans un second temps, pour chauffer la cuve d'émulsification grâce à la programmation de la rampe de température.

La présente invention a également pour objet des microparticules susceptibles d'être obtenues par le procédé décrit précédemment. Les microparticules obtenues sont de type matriciel, c'est-à-dire que les molécules bioactives sont dispersées dans une matrice de protéines laitières. Leur réseau protéique est compact et bien structuré.

Les microparticules obtenues selon le procédé de l'invention sont des microparticules non hydrosolubles et ne se réhydratent pas quand elles sont placées dans un milieu aqueux.

La taille, mais également la forme et la texture des microparticules sont des facteurs qui affectent la perception par un consommateur. En effet, des microparticules petites, sphériques et molles seront moins facilement détectées que des microparticules dures, rugueuses et de grande taille. La nature de la matrice alimentaire dans laquelle elles sont introduites est également déterminante. Par exemple, les mêmes microparticules seront plus facilement détectables par le consommateur si elles sont introduites dans un yaourt brassé plutôt que dans un yaourt ferme.

Conformément à l'invention, les microparticules peuvent être utilisées dans des compositions cosmétiques, pharmaceutiques ou alimentaires. A cette fin, elles peuvent être utilisées soit directement, soit sous forme d'une poudre obtenue après séchage desdites microparticules. Cette poudre et les compositions cosmétiques, pharmaceutiques et alimentaires qui les contiennent font également partie de l'invention.

Elles peuvent également être utilisées comme véhicule des molécules bioactives qu'elles contiennent.

En fonction du type d'application, c'est-à-dire selon la nature du produit dans lequel on souhaite ajouter les microparticules, la présente invention permet, en modifiant soit la vitesse d'agitation ou la nature du mobile d'agitation :

- de moduler la quantité de molécule bioactive encapsulée,
- d'ajuster la taille des microparticules et
- d'ajuster la forme des microparticules.

L'homme du métier, saura, à la lumière de ses connaissances générales adapter ces différents paramètres.

Les figures 1 à 5 et les exemples 1 à 3 qui suivent illustrent l'invention.

La figure 1 est une représentation schématique d'un exemple de dispositif apte à mettre en oeuvre le procédé selon l'invention. (101) enceinte fermée pour la réaction enzymatique, (102) enceinte fermée dite d'émulsification, (103) moyens qui permettent d'amener la solution de protéines obtenues à l'étape a) dans l'enceinte (102), (104) moyens de régulation de température.

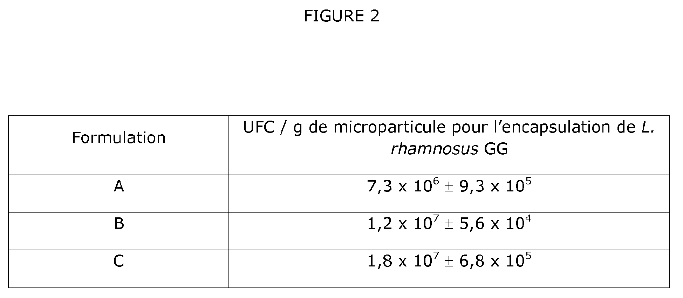

La figure 2 représente le nombre d'UFC (Unité formant colonie) contenu dans 1g de microparticules pour l'encapsulation de *L. rhamnosus* GG obtenu conformément à l'exemple 1.1.5. A : solution de caséine micellaire seule ; B : solution de caséine micellaire 90 % + protéines laitières solubles natives 10 % ; C : solution de caséine micellaire 90 % + protéines laitières solubles dénaturées 10 % ;

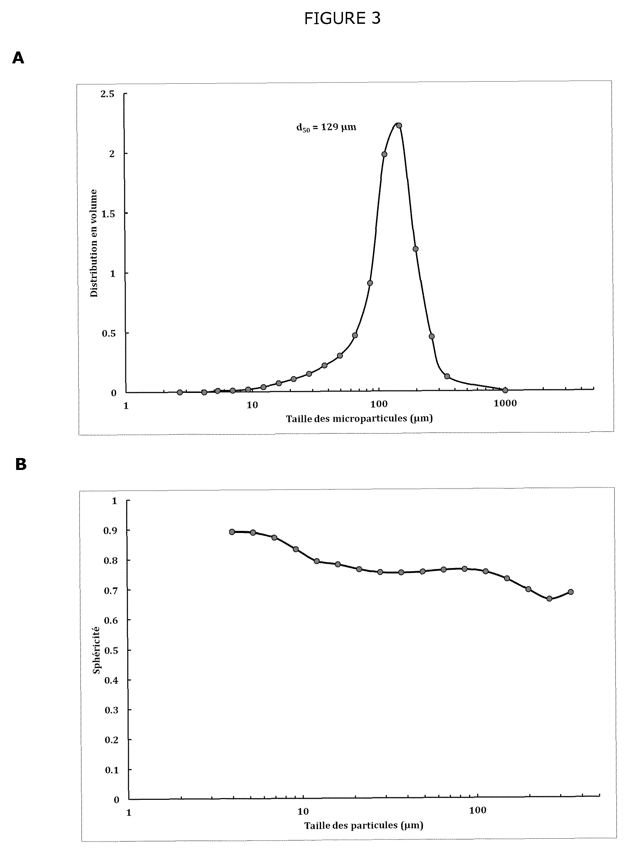

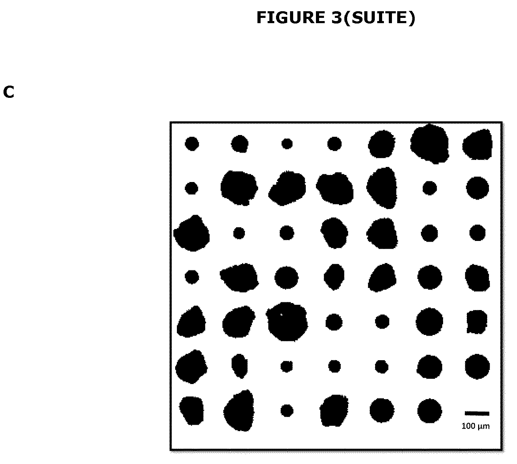

La figure 3A illustre la distribution des tailles de microparticules contenant *L. rhamnosus* GG obtenues selon le procédé de l'exemple 1.1.5 ; la figure 3B représente la sphéricité des microparticules en fonction de leur taille ; la figure 3C représente les microparticules obtenues selon le procédé de l'exemple 1.1.5 (image obtenue grâce à la caméra présente dans le granulomorphomètre). La matrice utilisée pour l'encapsulation de *L. rhamnosus* GG présentée dans cet exemple est composée de 90% de caséines micelllaires et 10% de protéines laitières solubles dénaturées (Formulation C de la figure 2).

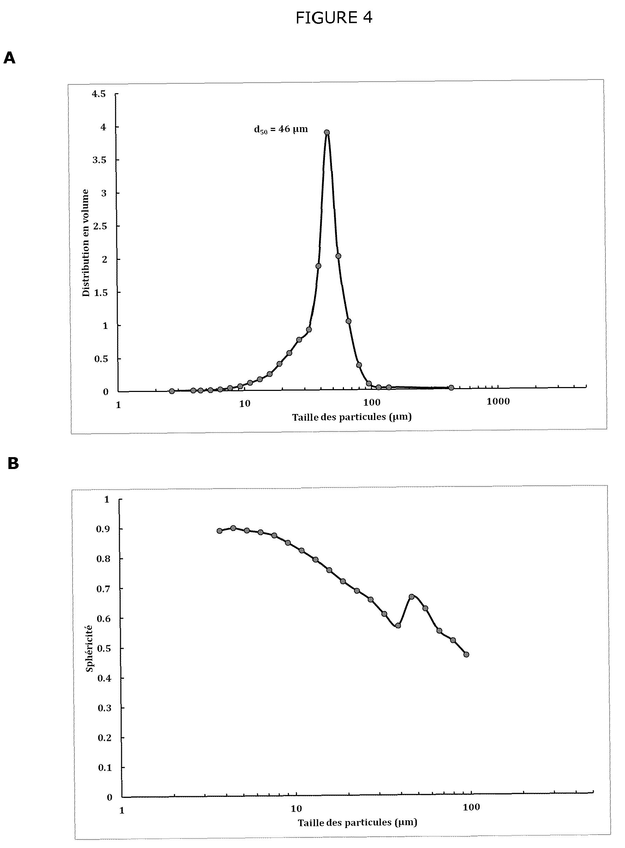

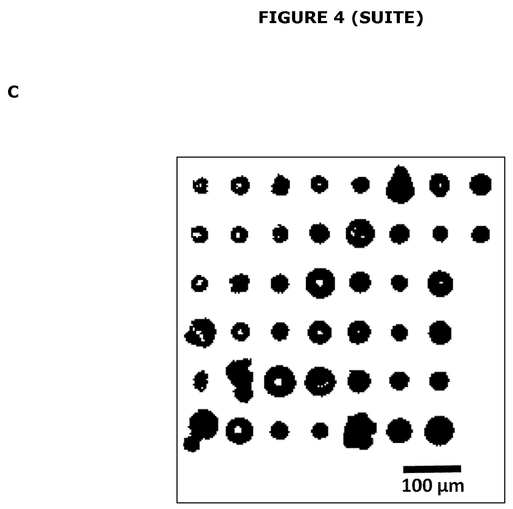

La figure 4A illustre la distribution des tailles de microparticules obtenues pour l'encapsulation de la quercétine selon le procédé de l'exemple 2 ; la figure 4B représente la sphéricité des microparticules en fonction de leur taille ; la figure 4C représente les microparticules obtenues selon le procédé de l'exemple 2. La matrice utilisée pour l'encapsulation de la quercétine est composée uniquement de caséines micellaires (Formulation A de la figure 2).

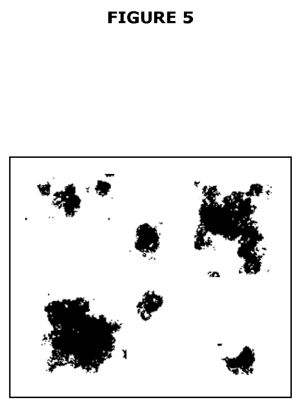

La figure 5 illustre les résultats d'essais d'encapsulation réalisés suivant le procédé de l'exemple 1. La matrice utilisée pour l'encapsulation des probiotiques est composée uniquement de lait écrémé et représente donc l'état antérieur de la technique.

**Exemple 1 : Microencapsulation de *L. rhamnosus* GG *dans différentes matrices protéiques d'origine laitière***

*1.1 Matériel et méthodes*

1.1.1. Préparation de la caséine micellaire

De la caséine micellaire est réhydratée dans l'eau pendant 2 heures à 25 °C sous agitation et puis laissée une nuit sous agitation pour donner une solution finale à une concentration de 12,5 % en caséine micellaire.

1.1.2. Préparation de la solution de protéines laitières solubles natives

Les protéines laitières solubles natives sont réhydratées dans de l'eau pendant 2 heures à 25 °C sous agitation et puis laissées une nuit sous agitation pour donner une solution finale à 12,5 % en protéines natives.

1.1.3. Préparation de la solution de protéines laitières solubles dénaturées :

La solution obtenue à l'étape précédente est chauffée pendant 10 minutes à 78 °C. La solution passe de translucide à opaque, signe de dénaturation protéique. Cette température de 78°C pendant 10 minutes pour un volume de solution de 200 ml permet d'obtenir une diminution de plus de 50% de structures secondaires (hélices alpha, boucles de grande taille (large loop), coudes (turn)).

1.1.4. Préparation des matrices protéiques

200 ml de solutions de protéines laitières sont préparés selon le tableau suivant :

| Formulations | Caséine micellaire (%) | Protéines solubles natives (%) | Protéines solubles dénaturées (%) | *L. rhamnosus* GG (ATCC 53103) (g pour 200 ml de solution protéique) |
|---|---|---|---|---|
| A | 100 | 0 | 0 | 0,50 |
| B | 90 | 10 | 0 | 0,50 |
| C | 90 | 0 | 10 | 0,50 |

1.1.5. Préparation d'une matrice à base de lait écrémé

Une solution à base de lait écrémé à 35 % est utilisée pour l'encapsulation de *L. rhamnosus* GG (ATCC 53103) comme référence de l'état antérieur de la technique.

1.1.6. Encapsulation

200 ml de formulations A, B ou C telles que préparées à l'étape précédente, ou 200 ml d'une solution à 35 % de lait écrémé, sont mis en contact, pendant 30 minutes, sous une agitation de 150 rpm (Lightin® LabMaster), avec la chymosine (CHY-MAX® de CHR Hansen) à raison de 0,18 IMCU/mL de lait, dans une enceinte fermée thermostatée (figure 1 (101)) réfrigérée à 5 °C grâce à un réfrigérant circulant dans la double paroi (figure 1 (104)).
La solution ainsi obtenue est ajoutée à un débit de 8,2 L/h à 800 ml d'huile de tournesol contenant 8 g de tween 80®, maintenu à une température de 5 °C grâce à un réfrigérant circulant dans la double paroi de la cuve d'émulsification (figure 1 (102)) d'un volume de 1500 ml et sous une agitation de 500 rpm (Heidolph Digital 2000 Bioblock Scientific). La fraction volumique $\varphi$ est de 0,2. L'agitation est maintenue pendant 10 minutes, de manière à former des gouttelettes, puis une rampe de température de 5 à 40 °C est appliquée en 25 minutes. Les gouttelettes liquides commencent à devenir solides autour de 21 °C. On obtient un système où des microparticules solides sont dispersées dans de la matière grasse.
L'huile excédentaire est éliminée par filtration. Les microparticules sont récupérées, lavées à l'eau distillée et séchées par lyophilisation.
Le nombre d'UFC (Unité Formant Colonie) contenu dans 1g de microparticules est déterminé par dénombrement microbiologique sur un milieu gélosé spécifique aux lactobacilles : le milieu MRS (de Man Rogosa and Sharpe).

1.1.7. Structures et distribution des tailles de microparticules

La distribution en taille et forme des microparticules est mesurée grâce à un granulo-morphomètre laser. La présence d'une caméra sur cet appareil permet également de visualiser le profil des microparticules.
La dispersion de taille de l'échantillon est évaluée en mesurant le span (s) qui norme la largeur de la distribution par rapport à la valeur médiane :

$$s = \frac{d_{90} - d_{10}}{d_{50}}$$

où

$d_{10}$ diamètre correspond à la ligne des 10% sur la courbe de distribution cumulée,

$d_{50}$ diamètre correspond à la ligne des 50% sur la courbe de distribution cumulée, et

$d_{90}$ diamètre correspond à la ligne des 90% sur la courbe de distribution cumulée,

Ainsi, les valeurs des diamètres $d_{10}$, $d_{50}$ et $d_{90}$ reflètent le fait que 10%, 50% et 90% de la population ont un diamètre inférieur à cette valeur.

La forme des microparticules étant un paramètre clé dans la sensorialité des produits contenant des microparticules, la sphéricité a été déterminée. Cette valeur peut varier entre 0 et 1, une valeur de 1 décrivant une microparticule parfaitement sphérique.

### 1.2 Résultats

#### 1.2.1. Taux d'encapsulation

Les résultats sont donnés dans le tableau de la figure 2.

Pour toutes les formulations testées, le taux d'encapsulation est très bon.

Les meilleurs résultats sont obtenus avec la formulation C avec 1,8 x $10^7$ UFC/g.

#### 1.2.2. Caractérisation des microparticules et distribution de la taille des microparticules

Les résultats sont donnés dans les figures 3A, 3B et 3C et la figure 5.

Les microparticules selon l'invention se présentent sous forme matricielle, c'est-à-dire qu'elles contiennent les bactéries dispersées dans la matrice protéique.

Elles sont bien individualisées sans agrégats et de forme sphériques (figure 3B) avec une sphéricité qui varie entre 0,7 et 0,9 en fonction de la taille des microparticules.

La distribution de la taille des microparticules est donnée dans la figure 3A. Les microparticules présentent un diamètre moyen de 129 μm. La valeur s est de 1,28 ± 0,02.

En revanche la matrice à base de lait écrémé présente une faible densité qui limite sa capacité de protection des molécules bioactives car elle ne résister pas aux conditions gastriques (figure 5).

### Exemple 2 : Microencapsulation de la quercétine dans différentes matrices protéiques d'origine laitière

#### 2.1 Matériel et méthodes

##### 2.2.1. Encapsulation

Le procédé d'encapsulation est le même que celui décrit à l'exemple 1. La quercétine est ajoutée au même moment que les bactéries probiotiques. La matrice utilisée est la suivante : 100% de caséines micellaires (formulation A de la figure 2). La quantité de quercétine est la suivante : 1% en poids/poids total de la formulation.

Les températures, vitesses d'agitation et temps utilisés sont exactement les mêmes que dans l'exemple 1.

#### 2.2 Résultats

##### 2.2.1. Taux d'encapsulation

Le taux d'encapsulation de cette biomolécule varie entre 78% et 86% selon la quantité initiale de quercétine rajoutée au départ dans le lait.

##### 2.2.2. Caractérisation des microparticules et distribution de la taille des microparticules

Ils sont donnés dans la figure 4.

Les microparticules se présentent sous forme matricielle. Elles sont bien individualisées sans agrégats et de forme sphériques (figure 4B) avec une sphéricité comprise entre 0,5 et 0,9.

La distribution de la taille des microparticules est donnée dans la figure 4A. Les microparticules présentent un diamètre moyen de 46 μm et une valeur de s de 0,97±0,15.

**Exemple 3 : Microencapsulation de _Carnobacterium maltaromaticum_ (LMA28) dans une formulation contenant des caséines et des protéines solubles dénaturées.**

L'encapsulation est réalisée selon le procédé décrit dans l'exemple 1 avec la solution C. Le taux d'encapsulation est de $10^5$ UFC/g.

Le procédé selon l'invention est transposable à l'échelle industrielle et les différents paramètres (vitesse d'agitation, nature du mobile d'agitation,...) peuvent être adaptés non seulement pour moduler la taille et la forme des microparticules mais également la quantité de molécule bioactive encapsulée.

**Revendications**

1.  Procédé d'encapsulation de molécules bioactives par des protéines laitières choisies dans le groupe comprenant :

    • de la caséine micellaire seule,
    • de la caséine micellaire associée à des protéines laitières solubles natives,
    • de la caséine micellaire associée à des protéines laitières solubles dénaturées, et
    • de la caséine micellaire associée à des protéines laitières solubles natives et à des protéines laitières solubles dénaturées,

    le dit procédé étant mis en oeuvre en circuit fermé et comprenant les étapes suivantes :

    a. la mise en contact d'une solution desdites protéines laitières présentant un pH compris entre 4,8 et 8 et contenant au moins une molécule bioactive à encapsuler, avec la chymosine, à une température comprise entre 1 et 10 °C, avantageusement 3 et 5 °C, jusqu'à ce qu'au moins 85 % du caséinomacropeptide soit clivé,
    b. l'addition progressive de la solution protéique obtenue à l'étape a) à une matière grasse en présence d'un agent tensioactif à une température comprise entre 1 et 10 °C, avantageusement entre 3 et 5°C sous agitation comprise entre 100 et 1400 rpm, jusqu' à formation de gouttelettes et obtention d'une émulsion,
    c. l'application d'une rampe de température allant de 1 à 60 °C, avantageusement de 5 à 40 °C, et
    d. une phase de séparation des microparticules formées.

2.  Procédé selon la revendication 1, **caractérisé en ce qu'**il comprend après l'étape d), une étape de récupération des microparticules formées et éventuellement une étape de séchage desdites microparticules, notamment une étape de lyophilisation.

3.  Procédé selon l'une quelconque des revendications précédentes **caractérisé en ce que** la matière grasse est choisie dans le groupe comprenant :

    • les huiles fluides entre 1 et 60 °C, en particulier des huiles végétales, notamment l'huile de tournesol,
    • la matière grasse laitière fractionnée liquide entre 1 et 60 °C et
    • les matières grasses bénéfiques pour la santé, notamment celles contenant des acides gras polyinsaturés.

4.  Procédé selon l'une quelconque des revendications précédentes **caractérisé en ce que** de la mucine est ajoutée aux protéines laitières.

5.  Procédé selon l'une quelconque des revendications précédentes **caractérisé en ce que** la chymosine est utilisée à une concentration comprise entre 0,01 et 0,5 IMCU/g de protéines laitières, avantageusement entre 0,05 à 0,4 IMCU/g de protéines laitières.

6.  Procédé selon l'une quelconque des revendications précédentes **caractérisé en ce que** dans la solution de protéines laitières, la caséine représente entre 70 et 100 % en poids de la solution, les protéines laitières solubles natives représentent entre 0 et 30 % en poids de la solution et les protéines laitières solubles dénaturées représentent entre 0 et 30 % en poids de la solution.

7.  Procédé selon l'une quelconque des revendications précédentes **caractérisé en ce que** la fraction volumique ($\varphi$)

$$\varphi = \frac{\text{Volume de protéine laitière}}{\text{Volume de protéine laitière} + \text{volume de matière grasse}}$$

est inférieure à 0,2.

8. Microparticules susceptibles d'être obtenues par le procédé selon l'une quelconque des revendications précédentes.

9. Utilisation des microparticules selon la revendication 8 dans des compositions cosmétiques, pharmaceutiques ou alimentaires.

10. Utilisation des microparticules selon la revendication 8 comme véhicule des molécules bioactives qu'elles contiennent.

**Patentansprüche**

1. Verfahren zur Verkapselung von bioaktiven Molekülen durch Milchproteine aus der folgenden Gruppe:

   • mizellares Kasein allein,
   • mizellares Kasein in Kombination mit nativen löslichen Milchproteinen,
   • mizellares Kasein in Kombination mit denaturierten löslichen Milchproteinen, und
   • mizellares Kasein in Kombination mit nativen löslichen Milchproteinen und denaturierten löslichen Milchproteinen,

   wobei das Verfahren im geschlossenen Kreislauf eingesetzt wird und folgende Schritte umfasst:

   a. die Kontaktierung einer Lösung der Milchproteine, welche einen pH-Wert zwischen 4,8 und 8 aufweist und mindestens ein zu verkapselndes, bioaktives Molekül enthält, mit dem Chymosin bei einer Temperatur zwischen 1-10 °C, vorzugsweise 3-5 °C, bis mindestens 85 % des Kaseinmakropeptids gespalten ist,
   b. die Zumischung in Stufen der im Schritt a) gewonnenen Proteinlösung zu einem Fett zusammen mit einem grenzflächenaktiven Stoff bei einer Temperatur zwischen 1-10 °C, vorzugsweise 3-5 °C, unter Rühren mit 100-1400 Umdrehungen pro Minute bis zur Tröpfchenbildung oder Gewinnung einer Lösung,
   c. die Anwendung einer Temperaturrampe von 1-60 °C, vorzugsweise 5-40 °C, sowie
   d. einen Schritt zur Trennung der gebildeten Mikropartikel.

2. Verfahren nach Anspruch 1, **dadurch gekennzeichnet, dass** es nach Schritt d) einen Schritt zur Rückgewinnung der gebildeten Mikropartikel und eventuell einen Schritt zur Trocknung dieser Mikropartikel, insbesondere einen Schritt der Gefriertrocknung, umfasst.

3. Verfahren nach einem der vorhergehenden Ansprüche, **dadurch gekennzeichnet, dass** das Fett aus der Gruppe ausgewählt wird, welche wie folgt umfasst:

   • die flüssigen Öle zwischen 1-60 °C, vor allem pflanzliche Öle, insbesondere Sonnenblumenöl,
   • das fraktionierte Milchfett zwischen 1-60 °C sowie
   • die gesundheitsfördernden Fette, insbesondere diejenigen, die mehrfach ungesättigte Fettsäure enthalten.

4. Verfahren nach einem der vorhergehenden Ansprüche, **dadurch gekennzeichnet, dass** den Milchproteinen Mucin zugemischt wird.

5. Verfahren nach einem der vorhergehenden Ansprüche, **dadurch gekennzeichnet, dass** das Chymosin in einer Konzentration von 0,01-0,5 IMCU/g an Milchproteinen, vorzugsweise 0,05-0,4 IMCU/g an Milchproteinen eingesetzt wird.

**6.** Verfahren nach einem der vorhergehenden Ansprüche, **dadurch gekennzeichnet, dass** in der Lösung der Milchproteine das Kasein zwischen 70-100 % vom Gewicht der Lösung ausmacht, die nativen löslichen Milchproteine zwischen 0-30 % vom Gewicht der Lösung ausmachen und die denaturierten löslichen Milchproteine zwischen 0-30 % vom Gewicht der Lösung ausmachen.

**7.** Verfahren nach einem der vorhergehenden Ansprüche, **dadurch gekennzeichnet, dass** der Volumenanteil (φ)

$$\varphi \; = \; \frac{\text{Volumen des Milchproteins}}{\text{Volumen des Milchproteins} + \text{Volumen des Fettes}}$$

unter 0,2 liegt.

**8.** Mikropartikel, gewinnbar durch das Verfahren nach einem der vorhergehenden Ansprüche.

**9.** Anwendung der Mikropartikel nach Anspruch 8 in Kosmetik-, Pharma- bzw. Lebensmittelzusammensetzungen.

**10.** Anwendung der Mikropartikel nach Anspruch 8 als Vehikel der bioaktiven Molekülen, die sie enthalten.

**Claims**

**1.** Method for encapsulating bioactive molecules with dairy proteins selected from the group comprising:

- micellar casein alone,
- micellar casein combined with native soluble dairy proteins,
- micellar casein combined with denatured soluble dairy proteins, and
- micellar casein combined with native soluble dairy proteins and with denatured soluble dairy proteins,

said method being implemented in a closed circuit and comprising the following steps:

a. bringing a solution of said dairy proteins having a pH comprised between 4.8 and 8 and containing at least one bioactive molecule to be encapsulated, in contact with chymosin, at a temperature comprised between 1 and 10°C, advantageously 3 and 5°C, until at least 85% of the caseinomacropeptide is cleaved,
b. the progressive addition of the protein solution obtained in step a) to a fat in the presence of a surfactant at a temperature comprised between 1 and 10°C, advantageously between 3 and 5°C under stirring comprised between 100 and 1400 rpm, until droplets are formed and an emulsion is obtained,
c. application of a temperature gradient ranging from 1 to 60°C, advantageously from 5 to 40°C, and
d. a phase of separating the microparticles formed.

**2.** Method according to claim 1, **characterized in that** it comprises after step d), a step of recovering the microparticles formed and optionally a step of drying said microparticles, in particular a lyophilization step.

**3.** Method according to any one of the preceding claims **characterized in that** the fat is selected from the group comprising:

- oils that are fluid between 1 and 60°C, in particular vegetable oils, in particular sunflower oil,
- fractionated dairy fat that is liquid between 1 and 60°C and
- fats that have health benefits, in particular those containing polyunsaturated fatty acids.

**4.** Method according to any one of the preceding claims **characterized in that** mucin is added to the dairy proteins.

**5.** Method according to any one of the preceding claims **characterized in that** the chymosin is used at a concentration comprised between 0.01 and 0.5 IMCU/g of dairy proteins, advantageously from 0.05 to 0.4 IMCU/g of dairy proteins.

6.  Method according to any one of the preceding claims **characterized in that** in the solution of dairy proteins, the casein represents between 70 and 100% by weight of the solution, the native soluble dairy proteins represent between 0 and 30% by weight of the solution and the denatured soluble dairy proteins represent between 0 and 30% by weight of the solution.

7.  Method according to any one of the preceding claims **characterized in that** the volume fraction (φ)

$$\varphi = \frac{\text{Volume of dairy protein}}{\text{Volume of dairy protein + volume of fat}}$$

is less than 0.2.

8.  Microparticles capable of being obtained by the method according to any one of the preceding claims.

9.  Use of microparticles according to claim 8 in cosmetic, pharmaceutical or food compositions.

10. Use of microparticles according to claim 8 as a vehicle for the bioactive molecules that they contain.

FIGURE 1

(103)

Moteur

**Pompe péristaltique**

Moteur

T°C

T°C

(102)

Emulsification

Réaction Enzymatique

Bain d'eau à circulation
• Maintien de la température froide
•Maintien de la température chaude

(101)

(104)

FIGURE 2

| Formulation | UFC / g de microparticule pour l'encapsulation de *L. rhamnosus* GG |
|:---:|:---:|
| A | $7,3 \times 10^6 \pm 9,3 \times 10^5$ |
| B | $1,2 \times 10^7 \pm 5,6 \times 10^4$ |
| C | $1,8 \times 10^7 \pm 6,8 \times 10^5$ |

FIGURE 3

A

B

**FIGURE 3(SUITE)**

C

100 μm

FIGURE 4

**A**

**B**

# FIGURE 4 (SUITE)

C

100 µm

**FIGURE 5**

## RÉFÉRENCES CITÉES DANS LA DESCRIPTION

**Documents brevets cités dans la description**

- EP 1876905 A

**Littérature non-brevet citée dans la description**

- **BURGAIN J. et al.** *Journal of Food Engineering,* 2011, vol. 104, 467-483
- **LIVNEY Y.D.** *Colloid & Interface Science,* 2010, vol. 15, 73-83
- **HEIDEBACH T. et al.** *International Dairy Journal,* 2009, vol. 19, 77-84
- **LISOVA et al.** *Czech Journal of Food Science,* 2013, vol. 31, 270-274